# EUROPEAN PATENT APPLICATION

(11) **EP 4 195 216 A1**
(43) Date of publication of application: **14.06.2023**
(21) Application number: 21213590.9
(22) Date of filing: 10.12.2021
(51) Int. Cl.: G16H 40/20, G16H 50/20, G16H 50/30, G06N 20/00, G06Q 10/00

(54) **AGENDA GENERATION SYSTEM**

(71) Applicant: Koninklijke Philips N.V., 5656 AG Eindhoven (NL)
(72) Inventor: VAN EE, Raymond, Eindhoven (NL); WESTERINK, Joanne Henriëtte Desirée Monique, Eindhoven (NL); MENA BENITO, Maria Estrella, Eindhoven (NL); LEUFKENS, Timmy Robertus Maria, Eindhoven (NL); WEIJSEN, Tim Elisabeth Joseph, Eindhoven (NL); PFUNDTNER, Stefan, Eindhoven (NL); TSONEVA, Tsvetomira Kirova, Eindhoven (NL)
(74) Representative: Philips Intellectual Property & Standards

(57) **Abstract**

The present invention relates to an agenda generation system (10), comprising:
- at least one first sensor unit (20); an input unit (30); a processing unit (40); and at least one output unit (50);
wherein the at least one first sensor unit is configured to acquire physiological data of a subject over an extended period of time prior to a current time point and provide the physiological data to the processing unit, wherein the physiological data is assigned to different times during the extended period of time, and wherein the physiological data comprises one or more of: heart rate, ventricular high rate, blood pressure, respiration rate, skin conductance, step count;
wherein the at least one first sensor unit is configured to acquire sleep data of the subject over the extended period of time and provide the sleep data to the processing unit, wherein the sleep data is assigned to different times during the extended period of time, and wherein the sleep data comprises one or more of: duration of sleep; total daily sleep duration, onset of sleep, quality of sleep,
wherein the input unit is configured to receive details of undertaken work data and undertaken leisure activity data of the subject over the extended period of time and provide the undertaken work data and undertaken leisure activity data to the processing unit, and wherein the undertaken work data and undertaken leisure activity data is assigned to different times during the extended period of time;
wherein the input unit is configured to receive details of planned work data and planned leisure activity data of the subject after the current time point and provide the planned work data and planned leisure activity data to the processing unit;
wherein the processing unit is configured to implement at least one trained machine learning algorithm to determine a sleep schedule comprising a planned onset and duration of sleep and/or determine a work schedule comprising scheduling of the planned work data and/or determine a leisure schedule comprising scheduling of the planned leisure activity data, and wherein the determination comprises analysis of: the physiological data, the sleep data, the undertaken work data, the undertaken leisure activity data, the planned work data, and the planned leisure activity data; and
wherein one or more of the at least one output unit is configured to output the sleep schedule and/or the work schedule and/or the leisure schedule.

## Description

### FIELD OF THE INVENTION

The present invention relates to an agenda generation system, an agenda generation method, as well as to a computer program element and a computer readable medium.

### BACKGROUND OF THE INVENTION

Shift work forces individuals to be awake and perform at a time when biological sleep propensity is high, and - conversely - they need to sleep when sleep propensity is minimal. It is very hard for night shift workers to get enough sleep during the day. They get a daily average of two to four hours less sleep than normal. It is hard for them to not fall asleep during the day.

This misalignment not only induces poor sleep and sleep problems, but may also cause health and physical symptoms. Misalignment of sleep/wake cycles or circadian timing system results in undesirable health consequences. Disturbed sleep is associated with anxiety, depression, and poor mental health. Another major negative effect of the disturbance of normal circadian cycle, is reflected in fatigue and reduction of energy level.

Working a shift schedule often means that a person is out of sync with the normal, daily life activities of (other) family members and friends. For family members and friends, it is difficult to connect to the shift worker and be aware of their general mental state and wellbeing. The normal way in which the members of a family pick up on changes in mood and stress of one of them, is not directly applicable to a shift worker. And problems are multiplied when two members of the family work in (uncoordinated) shifts. A consequence of being less in-sync with each other is that the shift worker is less supported in a natural way: Nobody will say "you look tired, let me make you a coffee" if they are not aware of it.

Shift work plays a negative role in the worker's physical and mental health. Shift workers feel that they have little control over their routines for sleep, relaxation and well-being. Working in shifts additionally creates difficulties in family life and tends to restrict worker's social and relaxation activities.

Current solutions are mainly focused on facilitating sleep and/or to improve performance during night work. Little attention is focused on how individuals actually cope with shift work and his/her personal life, social life, leisure activities and wellbeing in general.

There is a need to resolve these issues.

### SUMMARY OF THE INVENTION

It would be advantageous to provide improved assistance to shift workers. The object of the present invention is solved with the subject matter of the independent claims, wherein further embodiments are incorporated in the dependent claims. It should be noted that the following described aspects and examples of the invention apply to the agenda generation system, the agenda generation method as well as to the computer program element and the computer readable medium.

In a first aspect, there is provided

An agenda generation system, comprising:
- at least one first sensor unit;
- an input unit;
- a processing unit; and
- at least one output unit.

The at least one first sensor unit is configured to acquire physiological data of a subject over an extended period of time prior to a current time point and provide the physiological data to the processing unit. The physiological data is assigned to different times during the extended period of time, and the physiological data comprises one or more of: heart rate, ventricular high rate, blood pressure, respiration rate, skin conductance, step count. The at least one first sensor unit is configured to acquire sleep data of the subject over the extended period of time and provide the sleep data to the processing unit. The sleep data is assigned to different times during the extended period of time, and the sleep data comprises one or more of: duration of sleep, total daily sleep duration, onset of sleep, quality of sleep. The input unit is configured to receive details of undertaken work data and undertaken leisure activity data of the subject over the extended period of time and provide the undertaken work data and undertaken leisure activity data to the processing unit. The undertaken work data and undertaken leisure activity data is assigned to different times during the extended period of time. The input unit is configured to receive details of planned work data and planned leisure activity data of the subject after the current time point and provide the planned work data and planned leisure activity data to the processing unit. The processing unit is configured to implement at least one trained machine learning algorithm to determine a sleep schedule comprising a planned onset and duration of sleep and/or determine a work schedule comprising scheduling of the planned work data and/or determine a leisure schedule comprising scheduling of the planned leisure activity data. The determination comprises analysis of: the physiological data, the sleep data, the undertaken work data, the undertaken leisure activity data, the planned work data, and the planned leisure activity data. One or more of the at least one output unit is configured to output the sleep schedule and/or the work schedule and/or the leisure schedule.

In this way, a worker for example a shift worker, is provided with schedule information regarding when and for how long to sleep, when to carry out work task, and when and what leisure activities to undertake based upon the person's needs, how they have performed in the past, and their agendas.

It is to be note that ventricular high rate (VHR) relates to a heartbeat above some threshold, and this information can be assigned to different time (or moments) during the extended period of time.

In an example, the system comprises:
- at least one second sensor unit.

The at least one second sensor unit is configured to acquire further physiological data of one or more further subjects over the extended period of time prior and provide the further physiological data to the processing unit. The further physiological data is assigned to different times during the extended period of time, and the further physiological data comprises one or more of: heart rate, ventricular high rate, blood pressure, respiration rate, skin conductance, step count. The at least one second sensor unit is configured to acquire further sleep data of the one or more further subjects over the extended period of time and provide the further sleep data to the processing unit. The further sleep data is assigned to different times during the extended period of time, and the further sleep data comprises one or more of: duration of sleep; total daily sleep duration, onset of sleep, quality of sleep. The input unit is configured to receive details of undertaken school/work data and further undertaken leisure activity data of the one or more further subjects over the extended period of time and provide the undertaken school/work data and further undertaken leisure activity data to the processing unit. The undertaken school/work data and further undertaken leisure activity data is assigned to different times during the extended period of time. The input unit is configured to receive details of planned school/work data and further planned leisure activity data of the one or more further subjects after the current time point and provide the planned school/work data and further planned leisure activity data to the processing unit. The determination by the at least one trained machine learning algorithm of the sleep schedule and/or work schedule and/or leisure schedule comprises analysis of: the further physiological data, the further sleep data, the undertaken school/work data, the further undertaken leisure activity data, the planned school/work data, and the further planned leisure activity data.

In this way, the worker is provided with schedule information regarding when and for how long to sleep, when to carry out work task, and when and what leisure activities to undertake that takes into account other people such as family members.

This enables a shift worker's agenda to be generated taking into account children who are at school and/or other members of the family who work, Indeed, the agenda generation system can generate an agenda for the shift worker even if another family member is also a shift worker.

In an example, the determination of the work schedule comprises an identification of a conflict between a work item of the planned work data of the subject and a school/work item of the planned school/work data of the one or more further subjects.

In an example, the determination of the work schedule comprises a proposed re-scheduling of the work item and/or a proposed re-scheduling of the school/work item comprising utilization of the undertaken work data of the subject and the undertaken school/work data of the one or more further subjects.

In an example, the determination of the work schedule comprises an identification of a conflict between a work item of the planned work data of the subject and a leisure activity item of the planned leisure activity data of the one or more further subjects.

In an example, the determination of the work schedule comprises a proposed re-scheduling of the work item and/or a proposed re-scheduling of the leisure activity item comprising utilization of the undertaken work data of the subject and the undertaken leisure activity data of the one or more further subject.

In an example, the determination of the leisure schedule comprises an identification of a conflict between a leisure activity item of the planned leisure activity data of the subject and a school/work item of the planned school/work data of the one or more further subjects.

In an example, the determination of the leisure schedule comprises a proposed re-scheduling of the leisure activity item and/or a proposed re-scheduling of the school/work item comprising utilization of the undertaken leisure activity data of the subject and the undertaken school/work data of the one or more further subjects.

In an example, the determination of the leisure schedule comprises an identification of a conflict between a leisure activity item of the planned leisure activity data of the subject and a leisure activity item of the planned leisure activity data of the one or more further subjects.

In an example, the determination of the leisure schedule comprises a proposed re-scheduling of the leisure activity item of the subject and/or a proposed re-scheduling of the leisure activity item of the one or more further subjects comprising utilization of the undertaken leisure activity data of the subject and the undertaken leisure activity data of the one or more further subjects.

Thus, the system provides for conflict resolution with respect to their planned agenda and that of other people, such as family members that then provides an optimal solution that takes into account the give/take of these parties in the past.

In an example, the determination of the leisure schedule comprises an identification of a specific leisure activity item that does not occur often enough.

In an example, the determination of the leisure schedule comprises a scheduling of one or more time for undertaking of the special leisure activity item comprising utilization of the planned work activity data of the subject, the planned leisure activity data of the subject, the planned school/work data of the one or more further subjects, and the planned leisure activity data of the one or more further subjects.

Thus, the system determines that the person would benefit from undertaking specific leisure activities, and proposes times for such activities that takes into account their agenda and those of other people such as family members

In an example, the input unit is configured to receive first personal data of the subject and provide the first personal data to the processing unit. The first personal data comprises one or more of: age, weight, gender, height. The input unit is configured to receive second personal data of the subject and provide the second personal data to the processing unit. The second personal data comprises one or more of: perceived energy level of the subject, perceived fatigue level of the subject, perceived mood of the subject. The at least one first sensor is configured to acquire work related data during a latest work period prior to the current time point and provide the work related data to the processing unit and the work related data comprises one or more of: personal smartphone usage data, work phone usage data. The processing unit is configured to implement at least one trained machine learning algorithm to determine an energy score for the user. The determination comprises utilization of the physiological data of the subject during the latest work period, the first personal data, the second personal data, and the work related data during the latest work period; and wherein one or more of the at least one output unit is configured to output the energy score.

Thus, an energy score for the person is generated, that enables the person to be provided with an understanding of their energy level after having just finished work, and enables other people such as family members to also see this information and interact with the person as they would then like to do, such as offering to make the person an cup of coffee or generally comfort the person if their energy level was low.

In an example, the determination of the sleep schedule and/or the determination of the work schedule and/or the determination of the leisure schedule comprises utilization of the energy score.

In a second aspect, there is provided an agenda generation method, comprising:
acquiring by at least one first sensor unit physiological data of a subject over an extended period of time prior to a current time point and providing the physiological data to a processing unit, wherein the physiological data is assigned to different times during the extended period of time, and wherein the physiological data comprises one or more of: heart rate, ventricular high rate, blood pressure, respiration rate, skin conductance, step count;
acquiring by the at least one first sensor unit sleep data of the subject over the extended period of time and providing the sleep data to the processing unit, wherein the sleep data is assigned to different times during the extended period of time, and wherein the sleep data comprises one or more of: duration of sleep; total daily sleep duration, onset of sleep, quality of sleep,
receiving by an input unit details of undertaken work data and undertaken leisure activity data of the subject over the extended period of time and providing the undertaken work data and undertaken leisure activity data to the processing unit, and wherein the undertaken work data and undertaken leisure activity data is assigned to different times during the extended period of time;
receiving by the input unit details of planned work data and planned leisure activity data of the subject after the current time point and providing the planned work data and planned leisure activity data to the processing unit;
implementing by the processing unit at least one trained machine learning algorithm and determining by the at least one trained machine learning algorithm a sleep schedule comprising a planned onset and duration of sleep and/or determining by the at least one trained machine learning algorithm a work schedule comprising scheduling of the planned work data and/or determining by the at least one trained machine learning algorithm a leisure schedule comprising scheduling of the planned leisure activity data, and wherein the determining comprises analysis of: the physiological data, the sleep data, the undertaken work data, the undertaken leisure activity data, the planned work data, and the planned leisure activity data; and
outputting by one or more of at least one output unit the sleep schedule and/or the work schedule and/or the leisure schedule.

According to another aspect, there is provided a computer program element controlling one or more of the systems as previously described which, if the computer program element is executed by a processor, is adapted to perform the method as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawing:
Fig. 1 shows a schematic example of an agenda generation system;
Fig. 2 shows a representation of an agenda generation method; and
Fig. 3 shows a detailed example of an agenda generation system.

### DETAILED DESCRIPTION OF EMBODIMENTS

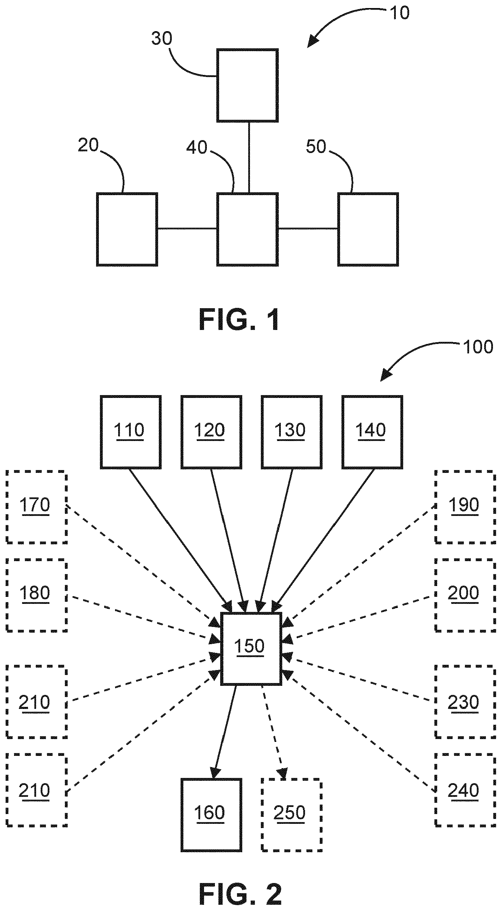

Fig. 1 shows an example of an agenda generation system 10. The system comprises at least one first sensor unit 20, an input unit 30, a processing unit 40, and at least one output unit 50. The at least one first sensor unit is configured to acquire physiological data of a subject over an extended period of time prior to a current time point and provide the physiological data to the processing unit. The physiological data is assigned to different times during the extended period of time, and the physiological data comprises one or more of: heart rate, ventricular high rate, blood pressure, respiration rate, skin conductance, step count. The at least one first sensor unit is configured to acquire sleep data of the subject over the extended period of time and provide the sleep data to the processing unit. The sleep data is assigned to different times during the extended period of time, and the sleep data comprises one or more of: duration of sleep; total daily sleep duration, onset of sleep, quality of sleep. The input unit is configured to receive details of undertaken work data and undertaken leisure activity data of the subject over the extended period of time and provide the undertaken work data and undertaken leisure activity data to the processing unit. The undertaken work data and undertaken leisure activity data is assigned to different times during the extended period of time (these can be the same different times to which the physiological data is assigned). The input unit is configured to receive details of planned work data and planned leisure activity data of the subject after the current time point and provide the planned work data and planned leisure activity data to the processing unit. The processing unit is configured to implement at least one trained machine learning algorithm to determine a sleep schedule comprising a planned onset and duration of sleep and/or determine a work schedule comprising scheduling of the planned work data and/or determine a leisure schedule comprising scheduling of the planned leisure activity data. The determination comprises analysis of: the physiological data, the sleep data, the undertaken work data, the undertaken leisure activity data, the planned work data, and the planned leisure activity data. One or more of the at least one output unit is configured to output the sleep schedule and/or the work schedule and/or the leisure schedule.

In an example, the sleep schedule and/or the work schedule and/or the leisure schedule is output on all of the at least one output units.

In an example an output unit can be a smart phone of the subject.

In an example, an output unit can be a smart phone of family members of the subject. In an example, an output unit can be a communal "board" in a house such as a tablet.

In an example, the at least one output unit can be all of a smart phone of the subject, a smart phone of family members of the subject, and a communal "board". But could be just two of these, such as a smart phone of the subject and a smart phone of family members of the subject or a smart phone of the subject and a communal board.

Thus, not only can the subject be made aware of outputs of the agenda generation system, but other family members can also be made aware of the outputs, enabling them to support the subject (such as a shift worker).

The agenda generation system can be a personal agenda generation system, generating an agenda for one person - for example a shift worker. But it can generate the agendas for more than one person, for example the shift worker and other family members and as such the agenda generation system could be considered to be a family agenda generation system.

According to an example, the system comprises at least one second sensor unit 60. The at least one second sensor unit is configured to acquire further physiological data of one or more further subjects over the extended period of time prior and provide the further physiological data to the processing unit. The further physiological data is assigned to different times during the extended period of time (the different times for the one or more further subjects can be the same different times to which the physiological data of the subject is assigned). The further physiological data comprises one or more of: heart rate, ventricular high rate, blood pressure, respiration rate, skin conductance, step count. The at least one second sensor unit is configured to acquire further sleep data of the one or more further subjects over the extended period of time and provide the further sleep data to the processing unit. The further sleep data is assigned to different times during the extended period of time (the different times can be the same different times to which the physiological data of the one or more further subjects is assigned). The further sleep data comprises one or more of: duration of sleep; total daily sleep duration, onset of sleep, quality of sleep. The input unit is configured to receive details of undertaken school/work data and further undertaken leisure activity data of the one or more further subjects over the extended period of time and provide the undertaken school/work data and further undertaken leisure activity data to the processing unit. The undertaken school/work data and further undertaken leisure activity data is assigned to different times during the extended period of time (the different times can be the same different times to which the physiological data of the one or more further subjects is assigned). The input unit is configured to receive details of planned school/work data and further planned leisure activity data of the one or more further subjects after the current time point and provide the planned school/work data and further planned leisure activity data to the processing unit. The determination by the at least one trained machine learning algorithm of the sleep schedule and/or work schedule and/or leisure schedule comprises analysis of: the further physiological data, the further sleep data, the undertaken school/work data, the further undertaken leisure activity data, the planned school/work data, and the further planned leisure activity data.

According to an example, the determination of the work schedule comprises an identification of a conflict between a work item of the planned work data of the subject and a school/work item of the planned school/work data of the one or more further subjects.

According to an example, the determination of the work schedule comprises a proposed re-scheduling of the work item and/or a proposed re-scheduling of the school/work item comprising utilization of the undertaken work data of the subject and the undertaken school/work data of the one or more further subjects.

According to an example, the determination of the work schedule comprises an identification of a conflict between a work item of the planned work data of the subject and a leisure activity item of the planned leisure activity data of the one or more further subjects.

According to an example, the determination of the work schedule comprises a proposed re-scheduling of the work item and/or a proposed re-scheduling of the leisure activity item comprising utilization of the undertaken work data of the subject and the undertaken leisure activity data of the one or more further subject.

According to an example, the determination of the leisure schedule comprises an identification of a conflict between a leisure activity item of the planned leisure activity data of the subject and a school/work item of the planned school/work data of the one or more further subjects.

According to an example, the determination of the leisure schedule comprises a proposed re-scheduling of the leisure activity item and/or a proposed re-scheduling of the school/work item comprising utilization of the undertaken leisure activity data of the subject and the undertaken school/work data of the one or more further subjects.

According to an example, the determination of the leisure schedule comprises an identification of a conflict between a leisure activity item of the planned leisure activity data of the subject and a leisure activity item of the planned leisure activity data of the one or more further subjects.

According to an example, the determination of the leisure schedule comprises a proposed re-scheduling of the leisure activity item of the subject and/or a proposed re-scheduling of the leisure activity item of the one or more further subjects comprising utilization of the undertaken leisure activity data of the subject and the undertaken leisure activity data of the one or more further subjects.

According to an example, the determination of the leisure schedule comprises an identification of a specific leisure activity item that does not occur often enough.

According to an example, the determination of the leisure schedule comprises a scheduling of one or more time for undertaking of the special leisure activity item comprising utilization of the planned work activity data of the subject, the planned leisure activity data of the subject, the planned school/work data of the one or more further subjects, and the planned leisure activity data of the one or more further subjects.

According to an example, the input unit is configured to receive first personal data of the subject and provide the first personal data to the processing unit. The first personal data comprises one or more of: age, weight, gender, height. The input unit is configured to receive second personal data of the subject and provide the second personal data to the processing unit. The second personal data comprises one or more of: perceived energy level of the subject, perceived fatigue level of the subject, perceived mood of the subject. The at least one first sensor is configured to acquire work related data during a latest work period prior to the current time point and provide the work related data to the processing unit. The work related data comprises one or more of: personal smartphone usage data, work phone usage data. The processing unit is configured to implement at least one trained machine learning algorithm to determine an energy score for the user, the determination comprising utilization of the physiological data of the subject during the latest work period, the first personal data, the second personal data, and the work related data during the latest work period. One or more of the at least one output unit is configured to output the energy score.

In an example, the energy score is output on a communal board, that is then in effect a family board. Thus everyone in the family is provided with information on the subject.

In an example, energy score is output on all of the at least one output unit.

According to an example, the determination of the sleep schedule and/or the determination of the work schedule and/or the determination of the leisure schedule comprises utilization of the energy score.

In an example, the processing unit is configured to continuously train the at least one machine learning algorithm on the basis of one or more of: sensor data received, input data received, and determinations made.

Fig. 2 An agenda generation method 100 in its basic steps, where optional steps are represented by dashed boxes and arrows. The method comprises:
acquiring 110 by at least one first sensor unit physiological data of a subject over an extended period of time prior to a current time point and providing the physiological data to a processing unit, wherein the physiological data is assigned to different times during the extended period of time, and wherein the physiological data comprises one or more of: heart rate, ventricular high rate, blood pressure, respiration rate, skin conductance, step count;
acquiring 120 by the at least one first sensor unit sleep data of the subject over the extended period of time and providing the sleep data to the processing unit, wherein the sleep data is assigned to different times during the extended period of time, and wherein the sleep data comprises one or more of: duration of sleep; total daily sleep duration, onset of sleep, quality of sleep,
receiving 130 by an input unit details of undertaken work data and undertaken leisure activity data of the subject over the extended period of time and providing the undertaken work data and undertaken leisure activity data to the processing unit, and wherein the undertaken work data and undertaken leisure activity data is assigned to different times during the extended period of time;
receiving 140 by the input unit details of planned work data and planned leisure activity data of the subject after the current time point and providing the planned work data and planned leisure activity data to the processing unit;
implementing 150 by the processing unit at least one trained machine learning algorithm and determining by the at least one trained machine learning algorithm a sleep schedule comprising a planned onset and duration of sleep and/or determining by the at least one trained machine learning algorithm a work schedule comprising scheduling of the planned work data and/or determining by the at least one trained machine learning algorithm a leisure schedule comprising scheduling of the planned leisure activity data, and wherein the determining comprises analysis of: the physiological data, the sleep data, the undertaken work data, the undertaken leisure activity data, the planned work data, and the planned leisure activity data; and
outputting 160 by one or more of at least one output unit the sleep schedule and/or the work schedule and/or the leisure schedule.

In an example, the method comprises:
acquiring 170 by at least one second sensor unit further physiological data of one or more further subjects over the extended period of time prior and providing the further physiological data to the processing unit, wherein the further physiological data is assigned to different times during the extended period of time, and wherein the further physiological data comprises one or more of:
   heart rate, ventricular high rate, blood pressure, respiration rate, skin conductance, step count;
acquiring 180 by the at least one second sensor unit further sleep data of the one or more further subjects over the extended period of time and providing the further sleep data to the processing unit, wherein the further sleep data is assigned to different times during the extended period of time, and wherein the further sleep data comprises one or more of: duration of sleep; total daily sleep duration, onset of sleep, quality of sleep,
receiving 190 by an input unit details of undertaken school/work data and further undertaken leisure activity data of the one or more further subjects over the extended period of time and providing the undertaken school/work data and further undertaken leisure activity data to the processing unit, and wherein the undertaken school/work data and further undertaken leisure activity data is assigned to different times during the extended period of time;
receiving 200 by the input unit is configured to receive details of planned school/work data and further planned leisure activity data of the one or more further subjects after the current time point and providing the planned school/work data and further planned leisure activity data to the processing unit;
wherein the determining by the at least one trained machine learning algorithm the sleep schedule and/or work schedule and/or leisure schedule comprises analyzing: the further physiological data, the further sleep data, the undertaken school/work data, the further undertaken leisure activity data, the planned school/work data, and the further planned leisure activity data.

In an example, the determining the work schedule comprises identifying a conflict between a work item of the planned work data of the subject and a school/work item of the planned school/work data of the one or more further subjects.

In an example, the determining the work schedule comprises a proposed re-scheduling of the work item and/or a proposed re-scheduling of the school/work item comprising utilizing the undertaken work data of the subject and the undertaken school/work data of the one or more further subjects.

In an example, the determining the work schedule comprises identifying a conflict between a work item of the planned work data of the subject and a leisure activity item of the planned leisure activity data of the one or more further subjects.

In an example, determining the work schedule comprises proposing a re-scheduling of the work item and/or proposing a re-scheduling of the leisure activity item comprising utilizing the undertaken work data of the subject and the undertaken leisure activity data of the one or more further subject.

In an example, the determining the leisure schedule comprises identifying a conflict between a leisure activity item of the planned leisure activity data of the subject and a school/work item of the planned school/work data of the one or more further subjects.

In an example, determining the leisure schedule comprises proposing a re-scheduling of the leisure activity item and/or proposing a re-scheduling of the school/work item comprising utilizing the undertaken leisure activity data of the subject and the undertaken school/work data of the one or more further subjects.

In an example, the determining the leisure schedule comprises identifying a conflict between a leisure activity item of the planned leisure activity data of the subject and a leisure activity item of the planned leisure activity data of the one or more further subjects.

In an example, determining the leisure schedule comprises proposing a re-scheduling of the leisure activity item of the subject and/or proposing a re-scheduling of the leisure activity item of the one or more further subjects comprising utilizing the undertaken leisure activity data of the subject and the undertaken leisure activity data of the one or more further subjects.

In an example, the determining the leisure schedule comprises identifying a specific leisure activity item that does not occur often enough.

In an example, the determining the leisure schedule comprises scheduling of one or more time for undertaking of the special leisure activity item comprising utilizing the planned work activity data of the subject, the planned leisure activity data of the subject, the planned school/work data of the one or more further subjects, and the planned leisure activity data of the one or more further subjects.

In an example, the method comprises:
receiving 210 by the input unit first personal data of the subject and providing the first personal data to the processing unit, wherein the first personal data comprises one or more of: age, weight, gender, height;
receiving 220 by the input unit second personal data of the subject and providing the second personal data to the processing unit, wherein the second personal data comprises one or more of: perceived energy level of the subject, perceived fatigue level of the subject, perceived mood of the subject;
acquiring 230 by the at least one first sensor work related data during a latest work period prior to the current time point and providing the work related data to the processing unit, and wherein the work related data comprises one or more of: personal smartphone usage data, work phone usage data;
implementing 240 by the processing unit at least one trained machine learning algorithm and determining by the at least one trained machine learning algorithm an energy score for the user, the determining comprising utilizing the physiological data of the subject during the latest work period, the first personal data, the second personal data, and the work related data during the latest work period; and
outputting 250 by one or more of the at least one output unit the energy score.

In an example, determining the sleep schedule and/or determining the work schedule and/or determining the leisure schedule comprises utilizing the energy score.

In an example, the processing unit is configured to continuously train the at least one machine learning algorithm on the basis of one or more of: sensor data received, input data received, and determinations made.

Thus, the new technique addresses the situation that shift workers face with respect to the consequences for the sleep and wake states, where misalignment of the circadian phase can result in undesirable consequences on sleep and as a consequence overall functioning. Disturbed sleep is reduced, which is also associated with anxiety, poor physical and mental health, and shift workers becoming desynchronized from their family's habits and routines is mitigated, leading to increased satisfaction with the amount of time spent with them. The new technique addresses how individuals actually cope with shift work and his/her personal life, social life, leisure activities and wellbeing in general. The new technique therefore provides an approach for assisting a shift worker, or multiple shift workers in a family, to schedule active and rest periods based upon the agendas, personal needs, personal levels of energy, and to create a better understanding of those needs within a family. This means that new methods and systems have been developed in the form of in effect a personalized sleep/rest/activity advisor, modeled around the shift worker's schedule, the schedule of the family, existing behavioral patterns, and self-defined goals. In this manner the new technique overcomes the current unmet needs by providing a tool to help a shift worker, or multiple shift workers in a family, to schedule active and rest periods based on personal needs and personal levels of energy, and to create a better understanding of those needs within a family. The result of that is optimal total daily sleep duration and sleep quality while maximizing the involvement in their social and family life. Additionally, the new approach provides a solution to augment energy levels and self-efficacy to increase time spent with family and friends and leisure activities in general.

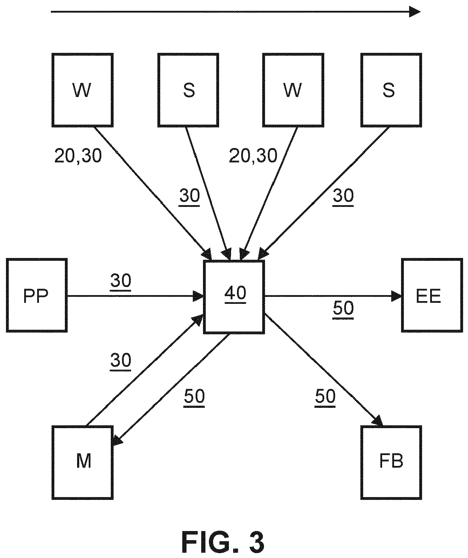

The new approach, as referred to above, is described in further specific detail, where reference is made to Fig. 3.

The new approach provides a method and system for assisting a shift worker, or multiple shift workers in a family, to schedule active and rest periods based upon the agendas, personal needs, personal levels of energy, and to create a better understanding of those needs within a family. Shift workers are supported in how to adapt and optimize their sleep, social, family, domestic life and leisure activities and well-being with their working schedules while actively involving their family members in this scheduling.

The new approach provides for personalization of sleep/rest/activity, modeled around the shift worker's schedule, the schedule of the family, existing behavioral patterns, and self-defined goals.

To obtain the desired balance the method and system of this new approach includes the following:
Assessing the features of the shift-worker: sleep data, physiological data, subjective data, work schedule, social activities
Assessing the features of other family members: sleep data, physiological data, subjective data, school/work schedule, social activities
Visualizing the information and sharing on a social, virtual, or domestic (mood) board
Quantifying the impact of shift-work on mental and physical capacity by means of an energy score
Selecting the optimal schedule and assisting in negotiation in agenda conflicts
The main elements of the new approach/technique consists of a system to manage and prioritize family related information, knowledge, insights, parameters and physiological signals in order to optimize the well-being of shift workers.

The system and method comprises the following elements:
A data input unit:
   Sensory and monitoring unit for collecting information:
   Objective information regarding the behavioral and/or lifestyle of the person. A sensor or series of sensors for monitoring objective behavioral (e.g. activity, counted steps, rest and sleep). This may be done by a wearable device
   Information related to Mood data from daytime physiological parameters;
Personal information /general data:
   Work schedule;
   Information concerning social moments;
   Self-defined preferences for specific timings or outcomes.
A processing unit:
   To acquire data from the data input unit (sensors and information/general data);
   To analyze and process the information.
A communication unit:
   User interface to provide the information (schedule advice) to the shift worker.

With reference to Fig. 3, a time line is shown at the top, where the shift worker undertakes work shifts identified as boxes with a "W" followed by periods of sleep identified as boxes with an "S". The shift worker can wear one or more sensor devices 20 and have access to an input device 30, such as a smart phone or tablet and which could also be accessed by other family members or they could have their own input devices and other family members could also wear one or more sensor devices 60. The sensor devices 20 monitor physiological parameters of the shift worker and monitor their sleeping condition and when they sleep and wake up. Via the input device 30 the shift worker can input information their work and leisure agendas and detail how they feel and information regarding their mood. This information is provided to a processing unit 40, and the agenda information is historical going into the past and also provides details on planned work and leisure activities. A personal profile indicated as "PP" can be input to the processing unit via the input unit, with information such as age, weight, gender, profession etc. This can be used to access a model from a database identified as "M", which is then provided to the processing unit. The model constitutes one or more machine learning algorithms, such as neural networks. The processing unit utilizes all the information it receives, which can be physiological parameters of the shift worker and of other family members and work/leisure agenda information (past and present) for the shift worker and family members, and determines an optimized work/leisure agenda for the shift worker, and also provides details on optimized sleep times. This information is fed back to the shift worker and family members, via an output unit 50, that could be the smart phone or tablet or other communication means, identified in Fig, 3 as the box "FB". The processing unit also outputs via the output unit 50 and energy score or estimation, identified as the box "EE", enabling the shift worker to visualize their current energy level, and importantly also enable other family members to assess how the shift worker feels at that moment in time. The processing unit, also updates the model on the basis of its processing and saves the model back to the database.

Several specific embodiments of the new technique/approach are now described.

In one embodiment, "learning unit" is implemented within the processing for the automatic identification of agenda conflicts that require negotiation between partners. The issues are raised to both parties with advice on possible choices and suggestions are made regarding the most optimal solution if one of the partners needs to give in, given what both partners have given/taken in the past. Here the term "learning unit" has been utilized to aid in the understanding of the functionality of the processing, however this "learning unit" is part of the model or machine learning algorithms as discussed above.

This embodiment also implements a "scheduling agent" to anticipate and prioritize relaxation events. The quality time of a relaxation event (for example go for a run in the woods) is important and eventually provides both individuals with energy. In order to be able to enjoy relaxation time, one should prioritize the relaxation moments. Especially, when a person did not engage in a relaxing event for a while, the algorithm prioritizes this to keep an optimal balance between work and leisure time. The scheduling agent detects 'important leisure activities', flags when such activity is not planned frequently enough, and indicates when they could take place taking both agendas into account. Here the term "scheduling" has been utilized to aid in the understanding of the functionality of the processing, however this "scheduling" is part of the model or machine learning algorithms as discussed above.

The learning unit uses information that is included to automatically adjust the proposed relaxation window(s) and activity windows. This process starts with a basic personalized preferred relaxation schedule, but includes more types of information as input parameters and *assigns dynamic weights* to them (priorities / norms).
adds priorities in seeing family, or hobby or career, and times at which these are preferred
adds the behavioral rhythm from the past predefined (n) weeks: (how much sleep? at what times? work at which times? family/hobby at which times? Individual sleep need? Level of restedness on specific mornings to automatically define the sleep need?)
adds info about morning/evening person.

The system then iterates towards optimum relaxation duration by automatically learning from outcome measures (Heart Rate, ventricular high rate (VHR), Blood pressure, respiration rate, etc) while maximizing family/social/hobby time.

It determines, given past behavioral rhythms and morningness/eveningness (chronotype) the likelihoods of success for a range of potential relaxation schedules, and then proposes the one with best chances of success. The system gradually learns to propose an optimal personalized, relaxation schedule.

Thus, in a specific example referring to Fig. 3 a number of inputs are provided to the central data analysis box or processing unit 40. One of these inputs can be a personal profile of the subject. This profile can be "personalized" by giving a weighting factors to each of the parameters that define the profile. Also, initial weighting factors can be assigned to each of the inputs coming from all input boxes leading to the processing unit 40. Reinforcement learning can then learn to assign updated weighting factors to the inputs so that there is less conflict in the output.

Another example relates to the situation where there are two shift workers in a family. As part of the agenda generation a period of time can be calculated that the two shift workers can be together, and then weighting factors can be assigned to possible activities if not all activities can be done. For example: One shift worker partner comes home at 8 in the evening and the other partner must leave at 4 in the morning. This gives 8 hours for possible activities. However once it is known that each of the partners needs 6 hours of sleep there is only 2 hours left for shared activities. Those can be filled by watching a movie, or doing a game, depending on the weighted preferences.

The system and method of this new approach utilizes an "additional unit" for determining and evaluating the energy level of the shift worker, or multiple shift workers within a family, but also the family members. Additionally, it provides an energy score/scale/index. This additional unit is again a part of the model or machine learning algorithms.

There are many factors that determine a person's energy level at the end of their shift. These range from the initial starting level, through actual workload during the shift, potential napping opportunities, etc. An easy to obtain metric (e.g. through unobtrusive wearable sensors) enables the guiding of further steps that lead to proper planning, increased consciousness, inform peers and family. Type of work per profession can make the descriptive (physiological) markers vastly different, making this not a trivial problem to solve. However, once the right markers are identified patterns can give insights into which days were "easy" and energy levels provide room for other activities, and which days were "tough" and sufficient sleep is a recommended necessity. The link between the multitude of potential markers to measure and an objective measure lie in other existing and validated metrics. Those can be sleep trackers or ways to gather subjective input (such as questionnaires).

Such information is obtained by means of analysis of the following parameters
Personal data: age, weight, gender, height,
Subjective data on perceived energy/fatigue, mood etc.
Shift tracker collecting objective data (wearable tracker, PPG, activity), smartphone usage data, work-related load metrics (e.g. # emergency calls at a call center)
Sleep tracker objectively measures sleep (optional)
Specific embodiments of sensor systems and shift worker interaction include:
   Wrist-worn HR(V) and activity tracker that collects shift data and provides objective sleep metrics, collects subjective user input and provides energy estimates through a smartphone application
   Somneo (Philips) wake-up light to setup alarms based on the personalized schedule of the users. Further, the integrated sensors in the Somneo are be used to derive more information on the user's behavior (bed timings).

The energy level, referred to above, is an energy score generated via implementation of a reinforcement learning system in the form of an energy estimation unit. This can be part of the model or machine learning algorithms referred to above, or a separate machine learning (e.g. neural network) environment. Initially an existing/default model or model based on demographic information (a generic user profile) is used. Over time the learning system adapts the output to optimize the prediction of energy levels. The reinforcement goal is to predict a high energy level if the amount of activity is high and sleep is minimally affected by tiredness, while a low energy level would result from low activity and sleep being highly affected e.g. with unintended naps and short sleep onset latency. Additionally, the energy score also reflects the subjective perception of the user and training will optionally also gear the model towards this.

Specific processing features:
Energy estimation unit: This trains and maintains (using AI reinforcement learning paradigms) a model that uses the shift tracker data to model energy based on demographic and user input unit data. It can then also detect patterns in the data, for instance that every Thursday morning after shiftwork the energy level is low. This modeled energy level is used for scheduling of (joint) activities, avoiding certain activities requiring much energy at moments that the energy level is forecasted to be low.

Model database: stores and gives access to existing models that form starting points for new users (e.g. existing models per profession)

Feedback unit:
Provides insight into which (optimal) behavior provides the best way to use/replenish available energy
Suggest activities based on energy level and - overtime - adjusts the advice to the most effective activities for the specific user using the learning engine.

Decline detection unit over time (months/years) can flag if physical and cognitive decline is detected, this can be used to tailor personalized advise, but also to prevent other risk groups from carrying out types of work that might accelerate their "decline" (e.g. flag type of work as unsuitable). This is again a part of the described model or machine learning algorithms.

It is to be noted that the current estimated level of energy can be used to provide advice with respect to upcoming event in agenda. The information may be used for a reorganization or rescheduling of the agenda. Also, the system enables to detect pattern in energy: e.g. on Thursday evenings there is always a low energy level and therefore no meetings should be planned. Furthermore, a Learning unit can be used, again a part of the model or machine learning algorithms. This unit gathers and analyzes information during a period of time, and the information is used to identify and establish recurrent patterns. Additionally, the information is used for further advice, such as agenda organization.

In another embodiment, the negotiation can be supported by a family mood and energy dashboard. On the mood & energy dashboard, the family members can visually learn how stressful or physically intense the past shifts have been and how much he/she has slept since. This information is presented to all family members, in order not to single out the shift worker.

This information is calculated based on the following input /information:
Daily Activity parameters: Step counter, stress (skin conductance, heart rate), energy level;
Sleep: sleep tracker, objective sleep parameters such as length and sleep onset moment, sleep quality
Subjective input: shift worker's self-reports on tiredness and mood as well as those of other family members, sleep satisfaction and a such provides input to the algorithm whether specific rated nights in the future should be prioritized or not, etc.

As described before, the method and system of this new approach utilizes a learning unit to be used for forecasting of the upcoming mood and energy states of all family members based on past patterns and recent data. The family mood & energy dashboard can be displayed on a screen in a central place in the house, e.g. on the fridge to share the data among family members, but it can also visualized on the smartphones of the family members.

Additionally, the system warns other family members when for one of them a steep decline in mood or energy is either noticed or predicted, so that they can make contact or take further action, despite the fact that they do not meet each other easily as a consequence of the shiftwork.

Furthermore, the system and method comprise a unit that provides advice to family members when the shift worker may be disturbed, or not to disturb him/her until a certain time, which is calculated based on (earlier) tracking inputs and known circadian rhythms.

It is to be noted that the output of the optimized work/leisure agenda, can be considered to be in effect a coaching feature to guide family behavior and activities: e.g. time to have a walk together, number of steps to be taken, or when not to disturb.

Thresholds can be assigned to provide minimal lengths of an advised relaxation period. For a person this may be 60 minutes. For another 15 minutes. The specific length of a needed relaxation period can be defined by the system based on prior events. Given a prior relaxation window of 20 minutes that was successful in reducing stress, but a window of 15m wasn't, the chance that a 20minute windows will be chosen for a feature event is higher than for a 15 minutes window.

The system and method also obtains alertness and safety data while driving, enabling as a measure of well-being to be determined from driving. However, the system can also detect that alertness is suboptimal for engaging into traffic, and a warning system will inform the user accordingly.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An agenda generation system (10), comprising:
- at least one first sensor unit (20); an input unit (30); a processing unit (40); and at least one output unit (50);
wherein the at least one first sensor unit is configured to acquire physiological data of a subject over an extended period of time prior to a current time point and provide the physiological data to the processing unit, wherein the physiological data is assigned to different times during the extended period of time, and wherein the physiological data comprises one or more of: heart rate, ventricular high rate, blood pressure, respiration rate, skin conductance, step count;
wherein the at least one first sensor unit is configured to acquire sleep data of the subject over the extended period of time and provide the sleep data to the processing unit, wherein the sleep data is assigned to different times during the extended period of time, and wherein the sleep data comprises one or more of: duration of sleep; total daily sleep duration, onset of sleep, quality of sleep,
wherein the input unit is configured to receive details of undertaken work data and undertaken leisure activity data of the subject over the extended period of time and provide the undertaken work data and undertaken leisure activity data to the processing unit, and wherein the undertaken work data and undertaken leisure activity data is assigned to different times during the extended period of time;
wherein the input unit is configured to receive details of planned work data and planned leisure activity data of the subject after the current time point and provide the planned work data and planned leisure activity data to the processing unit;
wherein the processing unit is configured to implement at least one trained machine learning algorithm to determine a sleep schedule comprising a planned onset and duration of sleep and/or determine a work schedule comprising scheduling of the planned work data and/or determine a leisure schedule comprising scheduling of the planned leisure activity data, and wherein the determination comprises analysis of: the physiological data, the sleep data, the undertaken work data, the undertaken leisure activity data, the planned work data, and the planned leisure activity data; and
wherein one or more of the at least one output unit is configured to output the sleep schedule and/or the work schedule and/or the leisure schedule.

2. System according to claim 1, the system comprising:
at least one second sensor unit (60);
wherein the at least one second sensor unit is configured to acquire further physiological data of one or more further subjects over the extended period of time prior and provide the further physiological data to the processing unit, wherein the further physiological data is assigned to different times during the extended period of time, and wherein the further physiological data comprises one or more of: heart rate, ventricular high rate, blood pressure, respiration rate, skin conductance, step count;
wherein the at least one second sensor unit is configured to acquire further sleep data of the one or more further subjects over the extended period of time and provide the further sleep data to the processing unit, wherein the further sleep data is assigned to different times during the extended period of time, and wherein the further sleep data comprises one or more of: duration of sleep; total daily sleep duration, onset of sleep, quality of sleep,
wherein the input unit is configured to receive details of undertaken school/work data and further undertaken leisure activity data of the one or more further subjects over the extended period of time and provide the undertaken school/work data and further undertaken leisure activity data to the processing unit, and wherein the undertaken school/work data and further undertaken leisure activity data is assigned to different times during the extended period of time;
wherein the input unit is configured to receive details of planned school/work data and further planned leisure activity data of the one or more further subjects after the current time point and provide the planned school/work data and further planned leisure activity data to the processing unit;
wherein the determination by the at least one trained machine learning algorithm of the sleep schedule and/or work schedule and/or leisure schedule comprises analysis of: the further physiological data, the further sleep data, the undertaken school/work data, the further undertaken leisure activity data, the planned school/work data, and the further planned leisure activity data.

3. System according to claim 2, wherein the determination of the work schedule comprises an identification of a conflict between a work item of the planned work data of the subject and a school/work item of the planned school/work data of the one or more further subjects.

4. System according to claim 3, wherein determination of the work schedule comprises a proposed re-scheduling of the work item and/or a proposed re-scheduling of the school/work item comprising utilization of the undertaken work data of the subject and the undertaken school/work data of the one or more further subjects.

5. System according to any of claims 2-4, wherein the determination of the work schedule comprises an identification of a conflict between a work item of the planned work data of the subject and a leisure activity item of the planned leisure activity data of the one or more further subjects.

6. System according to claim 5, wherein determination of the work schedule comprises a proposed re-scheduling of the work item and/or a proposed re-scheduling of the leisure activity item comprising utilization of the undertaken work data of the subject and the undertaken leisure activity data of the one or more further subject.

7. System according to any of claims 2-6, wherein the determination of the leisure schedule comprises an identification of a conflict between a leisure activity item of the planned leisure activity data of the subject and a school/work item of the planned school/work data of the one or more further subjects.

8. System according to claim 7, wherein determination of the leisure schedule comprises a proposed re-scheduling of the leisure activity item and/or a proposed re-scheduling of the school/work item comprising utilization of the undertaken leisure activity data of the subject and the undertaken school/work data of the one or more further subjects.

9. System according to any of claims 2-8, wherein the determination of the leisure schedule comprises an identification of a conflict between a leisure activity item of the planned leisure activity data of the subject and a leisure activity item of the planned leisure activity data of the one or more further subjects.

10. System according to claim 9, wherein determination of the leisure schedule comprises a proposed re-scheduling of the leisure activity item of the subject and/or a proposed re-scheduling of the leisure activity item of the one or more further subjects comprising utilization of the undertaken leisure activity data of the subject and the undertaken leisure activity data of the one or more further subjects.

11. System according to any of claims 2-10, wherein the determination of the leisure schedule comprises an identification of a specific leisure activity item that does not occur often enough.

12. System according to claim 11, wherein the determination of the leisure schedule comprises a scheduling of one or more time for undertaking of the special leisure activity item comprising utilization of the planned work activity data of the subject, the planned leisure activity data of the subject, the planned school/work data of the one or more further subjects, and the planned leisure activity data of the one or more further subjects.

13. System according to any of claims 1-12, wherein the input unit is configured to receive first personal data of the subject and provide the first personal data to the processing unit, wherein the first personal data comprises one or more of: age, weight, gender, height; wherein the input unit is configured to receive-second personal data of the subject and provide the second personal data to the processing unit, wherein the second personal data comprises one or more of: perceived energy level of the subject, perceived fatigue level of the subject, perceived mood of the subject; wherein the at least one first sensor is configured to acquire work related data during a latest work period prior to the current time point and provide the work related data to the processing unit, and wherein the work related data comprises one or more of: personal smartphone usage data, work phone usage data, and wherein the processing unit is configured to implement at least one trained machine learning algorithm to determine an energy score for the user, the determination comprising utilization of the physiological data of the subject during the latest work period, the first personal data, the second personal data, and the work related data during the latest work period; and wherein one or more of the at least one output unit is configured to output the energy score.

14. System according to claim 13, wherein determination of the sleep schedule and/or determination of the work schedule and/or determination of the leisure schedule comprises utilization of the energy score.

15. An agenda generation method (100), comprising:
acquiring (110) by at least one first sensor unit physiological data of a subject over an extended period of time prior to a current time point and providing the physiological data to a processing unit, wherein the physiological data is assigned to different times during the extended period of time, and wherein the physiological data comprises one or more of: heart rate, ventricular high rate, blood pressure, respiration rate, skin conductance, step count;
acquiring (120) by the at least one first sensor unit sleep data of the subject over the extended period of time and providing the sleep data to the processing unit, wherein the sleep data is assigned to different times during the extended period of time, and wherein the sleep data comprises one or more of: duration of sleep; total daily sleep duration, onset of sleep, quality of sleep,
receiving (130) by an input unit details of undertaken work data and undertaken leisure activity data of the subject over the extended period of time and providing the undertaken work data and undertaken leisure activity data to the processing unit, and wherein the undertaken work data and undertaken leisure activity data is assigned to different times during the extended period of time;
receiving (140) by the input unit details of planned work data and planned leisure activity data of the subject after the current time point and providing the planned work data and planned leisure activity data to the processing unit;
implementing (150) by the processing unit at least one trained machine learning algorithm and determining by the at least one trained machine learning algorithm a sleep schedule comprising a planned onset and duration of sleep and/or determining by the at least one trained machine learning algorithm a work schedule comprising scheduling of the planned work data and/or determining by the at least one trained machine learning algorithm a leisure schedule comprising scheduling of the planned leisure activity data, and wherein the determining comprises analysis of: the physiological data, the sleep data, the undertaken work data, the undertaken leisure activity data, the planned work data, and the planned leisure activity data; and
outputting (160) by one or more of at least one output unit the sleep schedule and/or the work schedule and/or the leisure schedule.
